# EUROPEAN PATENT SPECIFICATION

(11) **EP 2 730 274 B1**
(45) Date of publication and mention of the grant of the patent: **05.08.2015**
(21) Application number: 14154144.1
(22) Date of filing: 19.12.2011
(51) Int. Cl.: A61K 8/35, A61K 8/368, A61K 8/42, A61K 8/67, A61K 8/73, A61Q 5/06, A61Q 19/00, A61K 9/14

(54) **Instant powders for aqueous cosmetic and pharmaceutical applications**
Instantpulver für wässrige kosmetische und pharmazeutische Anwendungen
Poudres instantanées pour applications cosmétiques et pharmaceutiques aqueuses

(30) Priority: 21.12.2010 US 201061425343 P; 31.03.2011 EP 11160691
(43) Date of publication of application: 14.05.2014
(62) Divisional of application: 11797015.2
(73) Proprietor: Akzo Nobel Chemicals International B.V., 3811 MH Amersfoort (NL)
(72) Inventor: Thiewes, Sarah, Hammonton, NJ New Jersey 08037 (US); Tirol, Gloria Cansanay, Kendall Park, NJ New Jersey 08824 (US)
(74) Representative: Akzo Nobel IP Department

(56) References cited:
- EP-A1- 0 544 349
- US-A- 4 205 093
- US-A1- 2002 192 345
- US-B1- 6 667 060

## Description

### Field of Invention:

The invention relates to dry powder compositions for personal care and cosmetic applications. More specifically, the dry powder compositions comprise a blend of a cold water soluble starch and dehydro-xanthan as a naturally derived polymeric thickener and a cosmetically acceptable active.

### Background of the invention:

Liquid personal care products are ubiquitous and necessary in today's modern world. From shampoos to moisturizing lotions, bottles of such products can be found in medicine cabinet's across the globe. Active ingredients are commonly added to these products to obtain additional benefits such as anti-aging, anti-wrinkle and sun blocking properties. These active ingredients can and often are very sensitive to elevated temperatures (such as might be encountered in the shower or in a hot warehouse) or on exposure to sunlight.

An additional challenge of today's world is the travel restrictions limiting the amount and types of personal care products that may be carried through airport security. Liquids are typically limited to 100 ml plastic bottles and must be individually inspected. The ability to pack and carry powders that are easily reconstituted by the simple addition of water, would be a great benefit to many travelers. The difficulty in providing such products arises in that many powder formulations are difficult to reconstitute, and typically form lumps, fisheyes and non-uniformities when water is added without significant shear.

US 6 667 060 B1 (VANDECRUYS ROGER PETRUS GEREBE [BE] ET AL) 23 December 2003 (2003-12-23) discloses in Claim 10 a composition in the form of granules, comprising a cold water soluble starch. Xanthan gum is cited as excipient (column 9, line 19).

While the concept of having a dry powder containing all the essential ingredients of a cosmetic (such as a hand lotion) that can be converted into a usable product by simply adding water has been known for some time, the ability to obtain such a mixture and products including such mixtures that will have all the aesthetics of the liquid lotion or cream while limiting the negative attributes, such as non-uniformity, stickiness and heavy feel.

Thus, there still exists a need for a powdered product that can be readily and with minimum effort be reconstituted by adding water and mixed, for example, with a finger and the palm of the hand or by simply rubbing the hands together.

### Summary of the Invention

Accordingly, the invention relates to a personal care composition comprising a blend of a cold water soluble starch, a naturally derived polymeric thickener and a cosmetically or pharmaceutically acceptable active. Further, the composition is in the form of a solid.

### Description of the invention:

It has now been found that a blend of a pregelatinized modified starch and a naturally occurring thickening agent provides a composition, which when mixed with water can provide a smooth, uniform texture with acceptable viscosity and require low shear and quick dispersion times which are particularly well suited for in personal care and cosmetic applications. The advantages of a dry powder in these applications are ease of storage, increased stability and convenience of transport. The compositions exhibit the proper aesthetics with the ease of reconstituting the personal care formulation into an aqueous system. The powder system acts a both an instant thickening or gelling system and a carrier for the active ingredients.

Among the benefits for providing the ingredients in the powdered form includes the prevention of oxidation, such as with vitamin C (ascorbic acid). Oxidation of the active in the liquid form is much faster than in the solid form, and thus the solid form will have increased shelf life. Both solid and liquid actives can be utilized within the current invention. Thus, in an embodiment of this invention, the solid active will be mixed directly with the pregelatinized modified starch and the natural thickening agent.

In another embodiment, the active is a liquid. In such an embodiment a liquid can be absorbed onto a suitable support capable of high loading which provides a solid material that can then be mixed with the pregelatinized modified starch and naturally occurring thickener. In a further embodiment, the liquid active is an oil. When the liquid is absorbed onto a support, such as described above, the resulting powder or solid will contain a discrete liquid phase within the solid. The liquid phase can exist in the form of tiny droplets or a thin film on the interior walls of the support. The liquid active is not chemically converted to a solid form, but merely held in the support, for example, similar to a sponge, which upon addition of a suitable solvent (e.g. water) will release the liquid active.

For purposes of this invention, the support material used to make a high load dry solid from the liquid based active comprises a material that is able to absorb at least about 50% of its dry weight. In an embodiment, the support may be able to absorb at least 70% of its weight in the liquid.

The pregelatinized starches suitable for use in the present invention are those starches that have been treated with heat, moisture, or chemicals to disrupt the natural granular structure and render the starch soluble in water at below the gelatinization temperature of the native starch. For purposes of the invention, pregelatinized starches are also referred to as cold water soluble starches (CWS) and the terms are used interchangeably. For a general review of how to prepare pregelatinized starches see (Starch; Chemistry and Technology, R. L. Whistler, second edition, Academic Press, Inc. New York, 1984 pages 670 - 673). Additionally these products can be prepared by co-jet cooking coupled to a spray drier (see Kasica et al. US Patent# 5,571,552).

The starches of this invention can be derived form any source which typically yields starch. Some non-limiting examples of these starch types are corn, wheat, potato, rice, tapioca, sago, pea and sweet potato. The starches can be of the native variety or a hybrid variety produced by traditional breeding programs or by artificial gene manipulation. These hybrids include, but not limited to waxy versions (starches with little or no amylose) and high amylose cultivars. Waxy starches are typically defined as having less than about 5% amylose and sometime containing less than about 2% amylose. In an embodiment, the waxy starches have greater than 95% amylopectin. High amylose starches are defined as having greater than 40% amylose (with the exception of pea starch which has a high amylose content of greater than 27% amylose). In a further embodiment, the high amylose starches have an amylose content of greater than 60% amylose. In addition starches which have altered chain length and branch points are included in this application.

In addition to being pregelatinized the starches of this invention can further be modified to contain anionic, cationic, non-ionic and reactive groups. Derivatives of these types are described in "Modified Starches: Properties and Uses" O.B. Wurzburg, CRC Press Boca Raton, Florida, 1986 chapters 3-9. In an embodiment of this invention, the starch will contain non-ionic groups. In another embodiment the starch will be modified with a hydroxyalkyl group. In still another embodiment, the starch will be modified with propylene oxide (also known as hydroxypropylation).

The modified starches can be prepared in the granular form and then made CWS or can be reacted in solution and then recovered by means such as spray drying. The functionality of the starch will enhance the properties of the formulation and can provide many benefits. Some examples of benefits of modified starches used in the instant powder formulations include, but are not limited to, improved emulsion stability, foam structuring, creaminess, fragrance delivery, improved compatibility with other ingredients, improved adhesion to hair or skin, and the like. In addition degraded or fluidity starches will reduce the viscosity at a given solids, and are included as one of the modifications possible.

The second component of this invention is a polymeric thickener, which can be at least one synthetic polymeric thickener or gelling additive, at least one naturally derived polymeric thickener or gelling additive or combinations thereof. For purposes of this invention, a naturally derived polymer is defined as polymer that is isolated from a plant, microorganism or animal, and which may be used in non-modified form or which optionally may be subjected to chemical or physical modification Some examples of such materials include, but are not limited to, pectin, alginate, xanthan gum, proteins, guar gum, cellulosics and chemically or physically modified derivatives of these natural polymeric thickeners such as carboxymethyl cellulose, hydroxyethyl cellulose, hydroxyethyl ethylcellulose, hydroxypropyl cellulose, oxidized cellulose and dehydroxanthan gum. The natural polymer thickener is dehydro-xanthan gum.

Synthetic polymer thickeners are defined as any polymer that is prepared by a polymerization of monomers. Some non-limiting examples of synthetic thickeners are polyacrylic acid and cross-linked polyacrylic acid (carbomer), acrylates/steareth-20 itaconate copolymer, acrylates/ceteth-20 itaconate copolymer, acrylates/aminoacrylates/C 10-30 alkyl PEG-20 itaconate copolymer, acrylates/C 10-30 alkyl acrylate crosspolymer, polyacrylamide (and) C13-14 isoparaffin (and) laureth-7, acrylamides copolymer (and) mineral oil (and) C13-14 isoparaffin (and) polysorbate 85, hydroxyethylacrylate/sodium acrylol dimethyltaurate copolymer, and hydroxyethylacrylate/sodium acrylol dimethyltaurate copolymer.

The composition of the current invention is a blend of the starch and naturally occurring polymer thickener in a weight ratio of starch to polymer thickener of about 10:90 to about 90:10, and in another embodiment from about 20:80 to about 80:20 and in yet another embodiment from about 30:70 to 70:30. Within this range the blend is rapidly hydratable and will afford a uniform, consistent dispersion with minimal mixing. For example, minimal mixing can be the amount of mixing by a person mixing a small amount (e.g., generally about 5 to about 10 grams) of material in the palm of their hand with one or two fingers of the other hand.

In an embodiment of the invention, the personal care composition comprising the blend the cold water soluble starch, polymeric thickener and cosmetically or pharmaceutically acceptable active contains less than about 10 wt% water. In another embodiment, the composition contains less than about 7 wt% water, and in yet another embodiment less than about 5% water.

The CWS starch and naturally derived thickener can be provided as a solid or a powder. A powder is defined, for purposes of this invention, as a free flowing solid with a particle size of less than 1000 microns (average particle size distribution), which is approximately about the particle size of sand or smaller. The powders of this invention can be pressed or sintered into a solid form. Alternatively, if blend is formed as a solid, such as by extrusion or melt processing, it can be converted to a powder by grinding.

A wide variety of ingredient can be added to the blend of starch and natural thickener in order to provide a useful personal care formulation. Some examples of cosmetically or pharmaceutically acceptable actives that can be utilized in this invention include, but are not limited to, ascorbic acid, niacin, vitamin E (tocopherol), fish oil, fatty acids, such as stearic acid, oleic acid, t-butyl peroxide, collagen, surfactants, UV absorbing compounds, antibiotics and antifungal agents.

It is also important to the current invention that the formulations have the proper aesthetics. For example, fish eyes and lumps (non-uniformity in the hydration of the powder), grittiness and heavy oily feeling are undesirable. Accordingly, the powder containing the active will disperse in water by mixing the powder with water in the palm of the hand, and should be uniform and completely hydrated in less than about 30 seconds. In an embodiment of this invention, the powder will be substantially and uniformly dispersed in water with minimal mixing of the material in the palm of the hand for a period of about 10 to about 20 seconds. For purposes of this invention, minimal mixing is defined as the action gently rubbing the hands together, such as one would apply for a hand lotion. Another way to obtain minimal mixing is to use one or two fingers of one hand and gently mix the powder and water in the palm of the other hand. In an embodiment of the invention, the blend of the pregelatinized modified starch and the naturally occurring thickening agent are in the form of a powder. The rheological performance of the powder blends help to describe the properties in a systematic way that provides quantitative methods to the perception of the user. While each user may describe the feel or viscosity of each formulation a little differently, G' and G" provide details about the liquid (such as viscosity and cohesiveness) and gel (texture and strength) structure of the formulations. G' has been found to be suitable indicator of the feel of the formulation, in particular tackiness and heaviness of the lotion or crème. The smoothness of the formulation is applicable to G".

It has been found that G' of the formulation should be about 1300 pascals or less and the G" is about 170 pascals or less, as measured according to the Rheological Procedure described hereinbelow. In one embodiment of this invention G' is about 700 to about 1250 pascals. In another embodiment G" is from about 90 to 160 pascals. It should be recognized that the G' and G" of the formulation can be controlled somewhat separately by choosing the pregelatinized modified starch and the natural thickening agent to meet the needs for the particular application at hand. Inclusion of additional starches or thickening agents can help with control of the properties (e.g. viscosity, texture, speed to dissolve, etc.) for each specific application.

Depending on the type of personal care compositions, the viscosity of these formulations will vary. Methodologies for measuring viscosity of the various compositions include solution viscosity, such as measured by Brookfield (Brookfield Engineering Laboratories, Inc. Middleboro, Massachusetts, USA, 02346) viscometer or flow viscosity, such as measured with a glass capillary viscometer. Rheological methods for measuring viscosity using instruments such as a Rheometric Scientific rheometer (Rheometric Scientific Inc, Piscataway, NJ 08854) may also be used.

In an embodiment of the invention, the compositions of the instant invention can have a range of rheology viscosity measurement of from about 100 to about 1000 Pascal seconds at from about 10 to about 15% solids in water at 23°C. In another embodiment, the viscosity will be from about 300 to about 650 Pascal at about 14% solids at 23°C. In yet another embodiment, the viscosity of the formulation will be from about 350 to about 500 Pascal seconds at about 14% solids at 23°C.

In an embodiment, the active will be present in the dry formulation from about 1 percent to about 75 percent of the total dry weight of the blend (active plus CWS starch plus naturally derived thickener). In another embodiment, the active will be present form about 5 percent to about 50 percent of the total dry formula weight.

Depending on the application and viscosity required, in an embodiment, the amount of the powder blends (CWS starch blended with the natural derived thickener) will be from about 1 to about 50 percent based on the weight of the water to be added. In another embodiment, the powder blend will be from about 2 percent to about 25 percent of the amount of water to be added. In yet another embodiment, the amount of active is from about 5 percent to about 15 percent weight of the water to be added. The added water is that water that is used to hydrate the powder blend in formation of the useable product (e.g., crème or lotion).

The present invention will now be illustrated by the following examples. The examples are intended to exemplify the present invention but are not intended to limit the scope of the invention in any way. The breadth and scope of the invention are to be limited solely by the claims appended hereto.

### Experimental:

### Ingredients:

Dehydro-xanthan gum is sold as AMAZE™ XT and is available from Corn Products International, Bridgewater New Jersey.
Hydroxypropyl waxy starch phosphate is sold as STRUCTURE® XL and is available from Corn Products International, Bridgewater New Jersey.
Degraded, hydroxypropyl tapioca is sold as TEXTRA® PLUS and is available from Corn Products International, Bridgewater New Jersey.
Hydroxypropyl waxy starch is sold as ULTRASPERSE® HV and is available from Corn Products International, Bridgewater New Jersey.
CWS waxy is sold as ULTRASPERSE® A and is available from Corn Products International, Bridgewater New Jersey.

### MATERIALS AND METHODS and RESULTS/OBSERVATIONS

### Section 1

### Dry Blends

| INCI Designation | A1 | A2 | A3 | A4 | A5 | A6 | A7 | A8 |
|---|---|---|---|---|---|---|---|---|
| | %w/w | %w/w | %w/w | %w/w | %w/w | %w/w | %w/w | %w/w |
| Dehydroxanthan Gum | 75 | 50 | 75 | 75 | | | | |
| Hydroxypropyl waxy Starch Phosphate | | | | | 75 | 75 | 75 | 75 |
| Tocopherol Acetate | 25 | | | | 25 | | | |
| Ascorbic Acid | | 50 | | | | 25 | | |
| Glycerin | | | 25 | | | | 25 | |
| Hydroxyethyl urea | | | | 25 | | | | 25 |
| Total | 100 | 100 | 100 | 100 | 100 | 100 | 100 | 100 |

A5 to A8 are comparative examples.

### Blends with Water

| Ingredient Blend | Concentration | Features: |
|---|---|---|
| A1 plus water | 3% (1.5% vitamin E load) | Difficult to incorporate, fish eyes, stirring manually was better than shaking, too quick viscosity build, hazy |
| A2 plus water | 3% (1.5% vitamin C load) | Difficult to incorporate, fish eyes, stirring manually was better than shaking, too quick viscosity build, much clearer than with Vitamin E |
| A5 plus water | 10% (2.5% vitamin E load) | Provided instant thickening, but needed to add much more to achieve the same viscosity as Amaze XT, much more of a creamy gel like rheology |
| A6 plus water | 15% (3.75% vitamin C load) | Provided instant thickening, but needed to add much more to achieve the same viscosity as Amaze XT, much more of a creamy gel like rheology, needed to add more to build viscosity due to acidity of Vitamin C |

The glycerin and hydroxyethyl urea actives started to hydrate the Amaze™ XL/XT (dehydro-xanthan gum) so the results became like cake frosting.

### Section 2

Dry Blend: All powders were added into a jar. The lid was tightened on and the jar was shaken until uniform. Wet Out: The uniform dry blend was weighed into another jar and the water was added in. The lid was tightened on and the jar was shaken until no feeling of movement.

### Dry Blend

| **Dry Blend** | **B1** | **B2** | **B3** | **B4** | **B5** | **B6** | **B7** | **B8** | **B9** |
|---|---|---|---|---|---|---|---|---|---|
| Dehydro-xanthan gum | 5.25 | 13.13 | 21.00 | 6.00 | 15.00 | 24.00 | 6.75 | 16.88 | 27.00 |
| STRUCTURE XL | 15.75 | 7.88 | | 18.00 | 9.00 | | 20.25 | 10.13 | |
| ULTRASPERSE HV | 9.00 | 9.00 | 9.00 | 6.00 | 6.00 | 6.00 | 3.00 | 3.00 | 3.00 |
| | 30.00 | 30.00 | 30.00 | 30.00 | 30.00 | 30.00 | 30.00 | 30.00 | 30.00 |

| Results (5% solids in water) | | | | | | | | | |
|---|---|---|---|---|---|---|---|---|---|
| Viscosity Build | 2 | 1 | 3 | 3 | 2 | 2 | 3 | 1 | 1 |
| Fish Eyes | 2 | 3 | 4 | 2 | 3 | 4 | 2 | 4 | 4 |
| Wet Out Ratio | 3 | 5 | 5 | 3 | 5 | 5 | 3 | 5 | 5 |
| Feel | 2 | 1 | 1 | 1 | 2 | 2 | 3 | 2 | 2 |

The following is the criteria for evaluating the all the samples.

### Key

| Viscosity Build | 1(0-5s) | 2(5-10s) | 3(10-20s) | 4(20-30s) | 5(>30s) |
|---|---|---|---|---|---|
| Fish Eyes | 1-None | 2-Very Few | 3-Some | 4-Many | |
| Wet Out Ratio | 1-Low (Thin) | | 3-Good | | 5-High(Thick) |
| Feel | 1-Smooth & Cushion | 2-Smooth | 3-Slight slimy | 4-Slimy | 5-Gritty |

### Dry Blend

| **Dry Blend** | **C1** | **C2** | **C3** | **C4** | **C5** | **C6** | **C7** | **C8** | **C9** |
|---|---|---|---|---|---|---|---|---|---|
| Dehydro-xanthan gum | 5.25 | 13.13 | 21.00 | 6.00 | 15.00 | 24.00 | 6.75 | 16.88 | 27.00 |
| STRUCTURE XL | 15.75 | 7.88 | | 18.00 | 9.00 | | 20.25 | 10.13 | |
| ADVANTA-GEL P75 | 9.00 | 9.00 | 9.00 | 6.00 | 6.00 | 6.00 | 3.00 | 3.00 | 3.00 |
| | 30.00 | 30.00 | 30.00 | 30.00 | 30.00 | 30.00 | 30.00 | 30.00 | 30.00 |

| Results (5% solids in water) | | | | | | | | | |
|---|---|---|---|---|---|---|---|---|---|
| Viscosity Build | 3 | 2 | 3 | 4 | 3 | 1 | 4 | 2 | 1 |
| Fish Eyes | 3 | 4 | 4 | 3 | 4 | 4 | 2 | 3 | 4 |
| Wet Out Ratio | 1 | 3 | 5 | 1 | 3 | 5 | 3 | 5 | 5 |
| Feel | 3 | 1 | 1 | 1 | 3 | 1 | 1 | 1 | 1 |

### Dry Blend

| **Dry Blend** | **D1** | **D2** | **D3** | **D4** | **D5** |
|---|---|---|---|---|---|
| Dehydro-xanthan gum | 5.25 | 21.00 | 15.00 | 6.75 | 27.00 |
| STRUCTURE XL | 15.75 | | 9.00 | 20.25 | |
| TEXTRA PLUS | 9.00 | 9.00 | 6.00 | 3.00 | 3.00 |
| | 30.00 | 30.00 | 30.00 | 30.00 | 30.00 |

| Results (5% solids in water) | | | | | |
|---|---|---|---|---|---|
| Viscosity Build | 3 | 2 | 1 | 2 | 2 |
| Fish Fyes | 1 | 4 | 4 | 3 | 4 |
| Wet Out Ratio | 3 | 5 | 5 | 3 | 5 |
| Feel | 2 | 3 | 2 | 1 | 3 |

### Dry Blend

| **Dry Blend** | **E1** | **E2** | **E3** | **E4** | **E5** |
|---|---|---|---|---|---|
| Dehydro-xanthan gum | 5.25 | 21.00 | 15.00 | 6.75 | 27.00 |
| STRUCTURE XL | 15.75 | | 9.00 | 20.25 | |
| ULTRASPERSE A | 9.00 | 9.00 | 6.00 | 3.00 | 3.00 |
| | 30.00 | 30.00 | 30.00 | 30.00 | 30.00 |

| Results (5% solids in water) | | | | | |
|---|---|---|---|---|---|
| Viscosity Build | 3 | 2 | 1 | 3 | 1 |
| Fish Eyes | 2 | 4 | 4 | 3 | 4 |
| Wet Out Ratio | 1 | 3 | 5 | 3 | 5 |
| Feel | 2 | 2 | 1 | 1 | 3 |

ULTRASPERSE® A and TEXTRA PLUS® seemed to give the best overall formulations in combination with AMAZE™ XT (dehydro-xanthan gum) and STRUCTURE® XL.

### Section 3

Thirty-gram samples of each dry mix were prepared by weighing each powder into a 4oz short jar and mixed by hand until homogeneous. The dissolution characteristics of the dry powders were assessed by adding 2.5g of the test powder to a 4oz tall jar. Water (or aqueous test solution) was added to the 4oz tall jar (47.5g). The jar was shaken by hand and the changes observed over time.

### Dry Blend

| **Dry Blend** | **F1** | **F2** | **F3** | **F4** | **F5** | **F6** | **F7** | **F8** | **F9** |
|---|---|---|---|---|---|---|---|---|---|
| Dehydro-xanthan gum | 2.70 | 4.05 | 5.40 | 3.15 | 4.73 | 5.40 | 3.60 | 5.40 | 7.20 |
| STRUCTURE XL | 15.30 | 13.95 | 12.60 | 17.85 | 16.28 | 15.60 | 20.40 | 18.60 | 16.80 |
| ULTRASPERSE HV | 12.00 | 12.00 | 12.00 | 9.00 | 9.00 | 9.00 | 6.00 | 6.00 | 6.00 |
| | 30.00 | 30.00 | 30.00 | 30.00 | 30.00 | 30.00 | 30.00 | 30.00 | 30.00 |

| Results (5% solids in water) | | | | | | | | | |
|---|---|---|---|---|---|---|---|---|---|
| Viscosity Build | 3 | 4 | 4 | 3 | 2 | 2 | 3 | 3 | 2 |
| Fish Eyes | 2 | 4 | 4 | 2 | 1 to 2 | 1 | 1 | 4 | 4 |
| Wet Out Ratio | 3 | 4 | 3 | 3 | 4 | 4 | 4 | 3 | 4 |
| Feel | 2 | 2 | 1 | 2 | 1 | 2 | | | 1 |

### Dry Blend

| **Dry Blend** | **G1** | **G2** | **G3** | **G4** | **G5** | **G6** | **G7** | **G8** | **G9** |
|---|---|---|---|---|---|---|---|---|---|
| Dehydro-xanthan gum | 2.70 | 4.05 | 5.40 | 3.15 | 4.73 | 5.40 | 3.60 | 5.40 | 7.20 |
| STRUCTURE XL | 15.30 | 13.95 | 12.60 | 17.85 | 16.28 | 15.60 | 20.40 | 18.60 | 16.80 |
| TEXTRA PLUS | 12.00 | 12.00 | 12.00 | 9.00 | 9.00 | 9.00 | 6.00 | 6.00 | 6.00 |
| | 30.00 | 30.00 | 30.00 | 30.00 | 30.00 | 30.00 | 30.00 | 30.00 | 30.00 |

| Results (5% solids in water) | | | | | | | | | |
|---|---|---|---|---|---|---|---|---|---|
| Viscosity Build | 3 | 3 | 4 | 3 | 3 | 3 | 4 | 3 | 2 |
| Fish Eyes | 2 | 3 | 4 | 4 | 2 | 4 | 4 | 4 | 4 |
| Wet Out Ratio | 3 | 3 | 2 | 2 | 3 | | 2 | 3 | 4 |
| Feel | 2 | 2 | 2 | 2 | 2 | 2 | 2 | 2 | 2 |

### Section 4: Dry Blends with Actives

### Main Dry Blend

| **Dry Blend** | **H1** | **H2** | **H3** | **H4** | **H5** |
|---|---|---|---|---|---|
| AMAZE XT | 2.70 | 3.15 | 4.73 | 5.40 | 3.60 |
| STRUCTURE XL | 15.30 | 17.85 | 16.28 | 15.60 | 20.40 |
| ULTRASPERSE HV | 12.00 | 9.00 | 9.00 | 9.00 | 6.00 |
| | 30.00 | 30.00 | 30.00 | 30.00 | 30.00 |

| Results (5% solids in water) | | | | | |
|---|---|---|---|---|---|
| Viscosity Build | 4 | 3 | 3 | 3 | 2 |
| Fish Eyes | 1 | 1 | 2 | 4 | 2 |
| Wet Out Ratio | 3 | 3 | 4 | 3 | 3-4 |
| Feel | 2 | 2 | 2 | 2 | 2 |
| Potential Formula | Yes | Yes | Yes | No | Yes |

### Dihydroxyacetone (DHA)

| **Wet Out (5%)** | **I1** | **I2** | **I3** | **I4** | **I5** |
|---|---|---|---|---|---|
| Water | 47.50 | 47.50 | 47.50 | 47.50 | 47.50 |
| Blend Number | H1 | H2 | H3 | H4 | H5 |
| Dry Blend | 2.38 | 2.38 | 2.38 | 2.38 | 2.38 |
| DHA | 0.13 | 0.13 | 0.13 | 0.13 | 0.13 |
| | 50.00 | 50.00 | 50.00 | 50.00 | 50.00 |

Formulations degraded - not evaluated

The formulations could not be evaluated as they all degraded during preparation.

### Salicylic Acid

| **Wet Out (5%)** | **J1** | **J2** | **J3** | **J4** | **J5** |
|---|---|---|---|---|---|
| Water | 47.50 | 47.50 | 47.50 | 47.50 | 47.50 |
| Blend Number | H1 | H2 | H3 | H4 | H5 |
| Dry Blend | 2.38 | 2.38 | 2.38 | 2.38 | 2.38 |
| Salicylic Acid | 0.13 | 0.13 | 0.13 | 0.13 | 0.13 |
| | 50.00 | 50.00 | 50.00 | 50.00 | 50.00 |

| Results | | | | | |
|---|---|---|---|---|---|
| Viscosity Build | 5 | 3 | 3 | 2 | 2 |
| Fish Eyes | 4 | 4 | 4 | 4 | 3 |
| Wet Out Ratio | 1 | 2 | 3 | 4 | 1 |
| Feel | 2 | 2 | 2 | 2 | 2 |

### Hydroxyethyl Urea

| **Wet Out (5%)** | **K1** | **K2** | **K3** | **K4** | **K5** |
|---|---|---|---|---|---|
| Water | 47.50 | 47.50 | 47.50 | 47.50 | 47.50 |
| Blend Number | H1 | H2 | H3 | H4 | H5 |
| Dry Blend | 2.38 | 2.38 | 2.38 | 2.38 | 2.38 |
| HEU Powder | 0.13 | 0.13 | 0.13 | 0.13 | 0.13 |
| | 50.00 | 50.00 | 50.00 | 50.00 | 50.00 |

| Results | | | | | |
|---|---|---|---|---|---|
| Viscosity Build | 3 | 3 | 2 | 2 | 3 |
| Fish Eyes | 2 | 2 | 3 | 4 | 3 |
| Wet Out Ratio | 3-4 | 4 | 4 | 4 | 4 |
| Feel | Tacky | Tacky | Tacky | Tacky | Tacky |

### Dry Blends

| Dry Blend | **L1** | **L2** | **L3** | **L4** |
|---|---|---|---|---|
| Dehydro-xanthan gum | 1.50 | 1.50 | 1.50 | 7.20 |
| STRUCTURE XL | 16.50 | 19.50 | 22.50 | 13.80 |
| ULTRASPERSE HV | 12.00 | 9.00 | 6.00 | 9.00 |
| | 30.00 | 30.00 | 30.00 | 30.00 |

### Blends with Water

| **Wet Out (5%)** | | | | |
|---|---|---|---|---|
| Water | 47.5 | 47.5 | 47.5 | 47.5 |
| Dry Blend | 2.5 | 2.5 | 2.5 | 2.5 |
| | 50 | 50 | 50 | 50 |

| Results | | | | |
|---|---|---|---|---|
| Viscosity Build-Rating | 4 | 3 | 3 | 1 |
| Viscosity Build-Time | 22 | 17 | 15 | 5 |
| Fish Eyes | 2 | 2 | 2 | 4 |
| Wet Out Ratio | 2 | 2 | 2 | 4 |
| Feel | 2 | 2 | 2 | 2 |

### Dry Blends

| **Dry Blend** | **M1** | **M2** | **M3** |
|---|---|---|---|
| Dehydro-xanthan gum | 0.00 | 0.00 | 0.00 |
| STRUCTURE XL | 18.00 | 21.00 | 24.00 |
| ULTRASPERSE HV | 12.00 | 9.00 | 6.00 |
| | 30.00 | 30.00 | 30.00 |

### Blends with Water

| **Wet Out (5%)** | | | |
|---|---|---|---|
| Water | 47.5 | 47.5 | 47.5 |
| Dry Blend | 2.5 | 2.5 | 2.5 |
| | 50 | 50 | 50 |

| Results | | | |
|---|---|---|---|
| Viscosity Build-Ratinq | 5 | 5 | 5 |
| Viscosity Build-Time | 43 | 40 | 38 |
| Fish Eyes | 3 | 3 | 3 |
| Wet Out Ratio | 1 | 1 | 1 |
| Feel | 2 | 2 | 2 |

Ternary systems of dehydro-xanthan gum (AMAZE^{™} XT), hydroxypropyl waxy starch phosphate (STRUCTURE® XL), and degraded, hydroxypropyl tapioca (TEXTRA^{®} Plus) give unacceptable levels of fish-eye formation and therefore are no longer in consideration. Ternary systems of AMAZE^{™} XT, STRUCTURE® XL, and hydroxypropyl waxy starch (ULTRASPERSE^{®} HV) are able to achieve a potentially acceptable balance of key properties. STRUCTURE® XL should be <55% of the total dry powder composition. AMAZE^{™} XT should be 5-12% of the total dry powder composition. ULTRASPERSE^{®} HV acts as quick dissolving filler. STRUCTURE® XL contributes to tack.

All of the dry powder compositions are tacky during application and dry down on the skin. Once dried, the dry powder compositions give a smooth and appealing feel on the skin.

Active ingredients affect the formula differently in a case-by-case way. Vitamin C depresses viscosity and increased dissolution speed. Salicylic acid increases fish eye formation. Hydroxyethyl urea increases tack during application and drying.

### Section 4 - Vitamin C Formulations

Dry Blend: All powders were added into a jar. The lid was tightened on and the jar was shaken until uniform. Wet Out: Dry powder was added to the palm of the hand and the appropriate amount of water was then added. The two were mixed using one finger until a gel type consistency was formed.

### Dry Blends

| | N1 | N2 | N3 | N4 |
|---|---|---|---|---|
| AMAZE XT | 0.60 | 1.00 | 1.25 | 1.50 |
| STRUCTURE XL | 4.40 | 4.00 | 3.75 | 3.50 |
| Ascorbic Acid | 5.00 | 5.00 | 5.00 | 5.00 |
| | 10.00 | 10.00 | 10.00 | 10.00 |

### Dissolution in water

1:5 ratio, 0.5q dry powder
   A: Dissolution ok; tacky feel, slightly gummy feel
   B: Dissolution ok; not as tacky, not as gummy as A
   C: Slightly longer dissolution time; similar feel to B
   D: Very thick; not as tacky as B/C
1:8 ratio, 0.5g dry powder
   D: Slightly too much water; similar feel as 1:5D
1:6 ratio, 0.2g dry powder
   A: Good dissolution; thinner, slightly tackier than 1:6D
   D: Good ratio; similar tack and feel to 1:5D

### Dry Powder blended with Commercial Products

Lotion - Clean and Clear Morning Glo
   1:6 ratio
   D: Pills slightly while rubbing; no negative impact on feel
   A: Not as much pilling; no negative impact on feel
Serum - Moisture Repair
   1:6 ratio
   A Good dissolution, no negative impact on feel

### Dry formulation modified for a 1:6 ratio use level and 10% active

### Same ratio of XT to XL as D

| | grams | % |
|---|---|---|
| AMAZE XT | 1.20 | 12.00 |
| STRUCTURE XL | 2.80 | 28.00 |
| Ascorbic Acid | 6.00 | 60.00 |
| | 10.00 | 100.00 |

### Section 5 - Hair Fixative Formulations

Dry Blend: All powders were added into a jar. The lid was tightened on and the jar was shaken until uniform. Wet Out: Dry powder was added to a small weigh boat. The water was then added and the two were mixed by hand until a gel type consistency was formed.

### Dry Blends

| | **P1** | **P2** | **P3** | **P4** | **P5** |
|---|---|---|---|---|---|
| **Dry Phase** | grams | grams | grams | grams | grams |
| AMAZE XT | 0.50 | 0.75 | 1.00 | 1.25 | 1.50 |
| STRUCTURE XL | 7.50 | 7.25 | 7.00 | 6.75 | 6.50 |
| AMAZE® | 2.00 | 2.00 | 2.00 | 2.00 | 2.00 |
| | 10.00 | 10.00 | 10.00 | 10.00 | 10.00 |

### Results

| | | | | | |
|---|---|---|---|---|---|
| Fish Eyes | None | None | None | None | None |
| Visual Viscosity | Thin | Thin | Good | Th ick | Thick |

AMAZE® is a high amylose hydroxypropyl starch co-processed with polyvinyl pyrrolidinone and available from Corn Products International (Bridgewater, New Jersey)

### Dry Blends

| | **Q1** | **Q2** | **Q3** | **Q4** | **Q5** |
|---|---|---|---|---|---|
| **Dry Phase** | grams | grams | grams | grams | grams |
| Blend number | P1 | P2 | P3 | P4 | P5 |
| Amount | 8.0 | 8.0 | 8.0 | 8.0 | 8.0 |
| PVP | 2.00 | 2.00 | 2.00 | 2.00 | 2.00 |
| | 10.00 | 10.00 | 10.00 | 10.00 | 10.00 |

### Blends with Water

| **Wet Out** | | | | | |
|---|---|---|---|---|---|
| Dry Phase | 0.20 | 0.20 | 0.20 | 0.20 | 0.20 |
| Water | 5.00 | 5.00 | 5.00 | 5.00 | 5.00 |
| | 5.20 | 5.20 | 5.20 | 5.20 | 5.20 |

### Results

| | | | | |
|---|---|---|---|---|
| Fish Eyes | None | None | None | None |
| Visual Viscosity | Thin | Thin | Thin | Good |

Formulations P3 and Q4 and Q5 provided the best visual viscosity. All of the PVP version were thinner than their respective AMAZE formulations and took longer to gel.

### Section 7 - Rheology and Viscosity formulations

Formulations were prepared according to SOP - Standard procedure for Sample Preparation and Measurement Process of Rheological Properties of Instant Powders

### Rheological procedure:

The ascorbic acid was ground to insure that no big chunks were present and the particle size is in the same level as starch and dehydro-xanthan gum. Samples were screened to remove larger particles if necessary. The formulation was prepared by dry blending the ingredients in a small jar by shaking for 30 seconds. A Rheometric Scientific rheometer (model SR-5000 was set to the Power Dynamic Viscosity (RTS02) using the 25 mm parallel plates and a 2 mm gap. The frequency sweep range of 0.2rad/sec to 100 rad/sec and a 1% variation of strain were used for all tests. The sample was prepared by mixing 0.3g of the power with 1.8 g of distilled water on a glass plate using a glass rod. The sample was mixed vigorously until a uniform paste was obtained. The uniform paste should be achieved within 20sec. If not, a record of the time to achieve uniformity was recorded. The sample was stirred for an additional 10 sec, where the paste was immediately transferred into the rheometer and the test was started. The reading of G', G", tanδ, and viscosity at 2 rad/sec (shear rate) was recorded for each sample.

### Dry Blend - Ratios of %

| | **R1** | **R2** | **R3** | **R4** | **R5** | **R6** | **R7** |
|---|---|---|---|---|---|---|---|
| AMAZE XT™ | 0 | 10 | 20 | 30 | 40 | 60 | 100 |
| % STRUCTURE XL | 100 | 90 | 80 | 70 | 60 | 40 | 0 |
| G' (average, in pascals | 779.2 | 814.2 | 1149.8 | 1264.2 | 1634 | 1705 | 2993.7 |
| G" (average, in pascals) | 91.7 | 111.6 | 145 | 161.3 | 192.2 | 197.2 | 350.3 |
| Actual Viscosity (average, in pascal seconds) | 392.3 | 410.8 | 579.5 | 637.2 | 822.4 | 8582 | 1507.1 |

### Subjective Panel Study Results (R1 - R7)

Panelist were given the following instructions

Wash and dry hands. Place the pre-weighed powder in the palm of your hand. Add the pre-weighed water to the powder taking care not to lose any water. Begin mixing the powder and water together with one finger and count in seconds how long it takes the two to form a gel. Stop mixing once the gel has formed. Do not mix more than 30 seconds. Please rate using the scales below. Wash and dry hands between each sample.

### Scale Used

| | | | |
|---|---|---|---|
| Fish Eyes: | 1 - None | 3 - Few | 5 - Many |
| Visual Viscosity: | 1 - Too Thin | 3 - Just Right | 5 - Too Thick |
| Feel during rub in: | 1 - Not Tacky | 3 - Slightly Tacky | 5 - Tacky |
| | 1 - Light | | 5 - Heavy |
| | 1 - Smooth | | 5 - Gritty |

### Results

### Rub in time

| | Time |
|---|---|
| R1 | 11.75 |
| R2 | 11 |
| R3 | 14.875 |
| R4 | 16.5 |
| R5 | 13.25 |
| R6 | 15.375 |
| R7 | 12 |

### Fish Eyes and Visual Viscosity

| | Fish Eyes | Visual Viscosity |
|---|---|---|
| R1 | 1.375 | 3.25 |
| R2 | 2.125 | 3.375 |
| R3 | 2.25 | 3.625 |
| R4 | 3.375 | 3.375 |
| R5 | 4 | 3.875 |
| R6 | 4.375 | 3.625 |
| R7 | 4.75 | 4.25 |

### Feel

| | Feel Tack | Feel L--> H | Feel S --> G |
|---|---|---|---|
| R1 | 2.5 | 3.375 | 2.25 |
| R2 | 3.25 | 2.875 | 2.5 |
| R3 | 3 | 3.5 | 3.125 |
| R4 | 3.5 | 3.625 | 3.375 |
| R5 | 3.75 | 3.875 | 3.375 |
| R6 | 3.875 | 3.875 | 3 |
| R7 | 3.625 | 3.75 | 3.875 |

Based on the results from the panel test, the best samples for rub time (time of mixing by hand to form a gel) is sample R1 or R2. Sample R1 was also had the least amount of fish eyes. R2 had the best skin feel and provided a nice smooth texture.

The citation of any document is not to be construed as an admission that it is prior art with respect to the present invention.

While particular embodiments of the present invention have been illustrated and described herein, the invention is not intended to be limited to the details shown. Rather, various modifications may be made in the details within the range and scope of equivalents of the claims and without departing from the scope of the invention.

## Claims

1. A personal care composition comprising :
a blend of a cold water soluble starch; dehydro-xanthan gum as polymeric thickener; and a cosmetically or pharmaceutically acceptable active, wherein the composition is in the form of a solid.

2. The composition of claim 1 wherein the solid is in the form of a powder.

3. The composition of any one of the claims 1-2 wherein the active is in the form of a powder.

4. The composition of any one of the claims 1-3 wherein the active is a liquid.

5. The composition of claim 4 wherein the liquid is an oil.

6. The composition of claim 4 or 5 wherein the active is absorbed onto a support.

7. The composition of any one of the claims 1-6 wherein the starch contains greater than 95wt% amylopectin.

8. The composition of any one of the claims 1-7 wherein the starch is modified.

9. The composition of claim 8 wherein the starch is modified with at least one non-ionic group.

10. The composition of claim 9 wherein the modification is hydroxypropyl.

11. The composition of any one of the claims 1-10 wherein the blend has a rheology measurement of 100 to 1000 Pascal seconds at from 10 to 15% solids in water at 23°C.

12. The composition of any one of the claims 1-11 wherein the composition contains less than 10wt% water.

13. The composition of any one of the claims 1-12 wherein the starch and polymer thickener are present in a weight ratio of starch to polymer thickener in the range of 10:90 to 90:10.

## Patentansprüche

1. Körperpflegezusammensetzung umfassend:
eine Mischung aus einer in kaltem Wasser löslichen Stärke, Dehydroxanthan Gum als polymerisches Verdickungsmittel und einen kosmetisch oder pharmazeutisch akzeptablen Wirkstoff, wobei die Zusammensetzung die Form eines festen Stoffes hat.

2. Zusammensetzung nach Anspruch 1, wobei der feste Stoff die Form eines Pulvers hat.

3. Zusammensetzung nach einem der Ansprüche 1-2, wobei der Wirkstoff die Form eines Pulvers hat.

4. Zusammensetzung nach einem der Ansprüche 1-3, wobei der Wirkstoff eine Flüssigkeit ist.

5. Zusammensetzung nach Anspruch 4, wobei die Flüssigkeit ein Öl ist.

6. Zusammensetzung nach Anspruch 4 oder 5, wobei der Wirkstoff auf einem Träger aufgenommen wird.

7. Zusammensetzung nach einem der Ansprüche 1-6, wobei die Stärke mehr als 95 Gew.-% Amylopektin aufweist.

8. Zusammensetzung nach einem der Ansprüche 1-7, wobei die Stärke modifiziert ist.

9. Zusammensetzung nach Anspruch 8, wobei die Stärke mit mindestens einer nichtionischen Gruppe modifiziert ist.

10. Zusammensetzung nach Anspruch 9, wobei die Modifikation Hydroxypropyl ist.

11. Zusammensetzung nach einem der Ansprüche 1-10, wobei die Mischung eine rheologische Messung von 100 bis 1000 Pascalsekunden bei von 10 bis 15% Feststoffen in Wasser bei 23°C hat.

12. Zusammensetzung nach einem der Ansprüche 1-11, wobei die Zusammensetzung weniger als 10 Gew.-% Wasser enthält.

13. Zusammensetzung nach einem der Ansprüche 1-12, wobei die Stärke und das polymerische Verdickungsmittel in einem Gewichtsverhältnis von Stärke zu polymerischem Verdickungsmittel in dem Bereich von 10:90 bis 90:10 vorhanden sind.

## Revendications

1. Composition de soins personnels comprenant :
un mélange d'un amidon soluble dans l'eau froide ; d'une gomme de déshydroxanthane en tant qu'épaississant polymère ; et d'un ingrédient actif cosmétiquement ou pharmaceutiquement acceptable, où la composition est sous la forme d'un solide.

2. Composition de la revendication 1 dans laquelle le solide est sous la forme d'une poudre.

3. Composition de l'une quelconque des revendications 1 à 2 dans laquelle l'ingrédient actif est sous la forme d'une poudre.

4. Composition de l'une quelconque des revendications 1 à 3 dans laquelle l'ingrédient actif est un liquide.

5. Composition de la revendication 4 dans laquelle le liquide est une huile.

6. Composition de la revendication 4 ou 5 dans laquelle l'ingrédient actif est absorbé sur un support.

7. Composition de l'une quelconque des revendications 1 à 6 dans laquelle l'amidon contient plus de 95% en poids d'amylopectine.

8. Composition de l'une quelconque des revendications 1 à 7 dans laquelle l'amidon est modifié.

9. Composition de la revendication 8 dans laquelle l'amidon est modifié avec au moins un groupe non-ionique.

10. Composition de la revendication 9 dans laquelle la modification est de l'hydroxypropyle.

11. Composition de l'une quelconque des revendications 1 à 10 dans laquelle le mélange a une mesure de rhéologie de 100 à 1000 Pascal.secondes à une plage allant de 10 à 15% de solides dans l'eau à 23°C.

12. Composition de l'une quelconque des revendications 1 à 11 dans laquelle la composition contient moins de 10% en poids d'eau.

13. Composition de l'une quelconque des revendications 1 à 12 dans laquelle l'amidon et l'épaississant polymère sont présents dans un rapport pondéral entre l'amidon et l'épaississant polymère dans la plage allant de 10:90 à 90:10.
